# FASCICULE DE BREVET EUROPEEN

(11) **EP 1 250 042 B1**
(45) Date de publication et mention de la délivrance du brevet: **01.10.2003**
(21) Numéro de dépôt: 01907620.7
(22) Date de dépôt: 18.01.2001
(51) Int. Cl.: A01N 3/00, A01N 3/02, A01N 25/34, A01N 59/12, A23B 7/152, A23L 3/3427, B65D 81/26, B01D 53/02

(54) **DISPOSITIF DESTINE A LUTTER CONTRE LA DEGRADATION DES VEGETAUX APRES RECOLTE ET PROCEDE DE FABRICATION DUDIT DISPOSITIF**
VORRICHTUNG ZUR UNTERDRÜCKUNG DES VERDERBENS VON PFLANZEN NACH DER ERNTE UND VERFAHREN ZU IHRER HERSTELLUNG
DEVICE FOR PROTECTING PLANTS AGAINST POST-HARVEST DECAY AND METHOD FOR MAKING SAME

(30) Priorité: 28.01.2000 FR 0001103
(43) Date de publication de la demande: 23.10.2002
(73) Titulaire: Ahlstrom Research and Competence Center, 38780 Pont Eveque (FR)
(72) Inventeur: DUSSAUD, Joseph, F-38200 Vienne (FR); BONDUELLE, Laurent, F-75012 Paris (FR)
(74) Mandataire: Vuillermoz, Bruno
(86) Numéro de dépôt international: FR0100154
(87) Numéro de publication internationale: WO01054496

(56) Documents cités:
- EP-A- 0 071 533
- EP-A- 0 515 764
- WO-A-95/31093
- WO-A-99/05922
- US-A- 2 139 619
- US-A- 2 701 774
- US-A- 4 906 398
- DATABASE WPI Section Ch, Week 198921 Derwent Publications Ltd., London, GB; Class A14, AN 1989-154424 XP002151499 & JP 01 094982 A (SKYLITE IND), 13 avril 1989 (1989-04-13)
- PATENT ABSTRACTS OF JAPAN vol. 017, no. 158 (C-1041), 29 mars 1993 (1993-03-29) -& JP 04 320641 A (NIPPON KAYAKU CO LTD), 11 novembre 1992 (1992-11-11)
- PATENT ABSTRACTS OF JAPAN vol. 1999, no. 03, 31 mars 1999 (1999-03-31) -& JP 10 337802 A (HIRAI MASAO), 22 décembre 1998 (1998-12-22)
- PATENT ABSTRACTS OF JAPAN vol. 014, no. 517 (M-1047), 13 novembre 1990 (1990-11-13) -& JP 02 215527 A (AKIE TSURUIZUMI;OTHERS: 01), 28 août 1990 (1990-08-28)
- PATENT ABSTRACTS OF JAPAN vol. 013, no. 394 (C-631), 31 août 1989 (1989-08-31) -& JP 01 138243 A (ARAI BUSSAN KK), 31 mai 1989 (1989-05-31)
- PATENT ABSTRACTS OF JAPAN vol. 014, no. 315 (M-0995), 6 juillet 1990 (1990-07-06) & JP 02 106335 A (AKIE TSURUIZUMI;OTHERS: 01), 18 avril 1990 (1990-04-18)

## Description

L'invention concerné un dispositif destiné à lutter contre la dégradation des végétaux après récolte. Elle se rapporte également au procédé de fabrication dudit dispositif.

Dans la suite de la description et dans les revendications, par le terme « végétaux », on désigne toute matière végétale incluant de manière non limitative les fruits, légumes, plantes, fleurs...

Les phénomènes responsables de la dégradation des végétaux après leur récolte sont connus. Pour l'essentiel, les végétaux émettent un certain nombre de gaz qui stimulent eux-mêmes la production de gaz de sorte que le système est autoalimenté en permanence jusqu'à la dégradation complète du végétal. En outre, l'émission de gaz conduit à la formation de moisissures et au développement de bactéries. Parmi les gaz dégagés, figurent essentiellement le dioxyde de carbone et l'éthylène. Il a en outre été démontré que la présence de dioxyde de carbone tendait à diminuer l'émission d'éthylène.

Pour lutter contre la dégradation des végétaux après leur récolte, on a recherché différentes solutions visant à dégrader l'éthylène, par rupture de la double liaison de la molécule.

Ainsi, on a proposé dans le document US-A-4 235 750 d'adsorber l'éthylène dégagé par la plante sur une poudre d'alumine activée, puis d'oxyder l'éthylène ainsi fixée au moyen d'un agent oxydant du type permanganate de potassium. La présence d'eau étant nécessaire à la réaction d'oxydation, la composition décrite comprend en outre un gel de silice activée destiné à adsorber l'humidité présente dans l'atmosphère. En pratique, la poudre est introduite soit dans des sachets, à raison de 10 à 30 g par sachet, soit dans les nombreux compartiments de plaques rectangulaires, la plaque étant recouverte d'un filtre permettant le passage des gaz.

Dans le document EP-A-0 071 533, le gel de silice a été substitué par un sel hygroscopique du type chlorure de calcium. Le mélange de poudre obtenu est placé à raison de 20 g dans un sachet de toile de nylon perméable à l'air.

Les deux dispositifs décrits dans les documents précités présentent l'inconvénient de mettre en oeuvre une quantité de produit importante de l'ordre de 20 g en moyenne, conduisant à augmenter considérablement le coût du traitement.

De plus, lorsque les végétaux sont stockés, par exemple dans des cagettes, comme c'est le cas des fruits et légumes ou dans des containers, comme c'est le cas des fleurs, les dispositifs décrits, compte tenu de leur encombrement, ne peuvent être positionnés qu'à proximité d'un certain nombre d'entre eux. Il s'ensuit que les végétaux ne sont pas conservés de façon uniforme.

En outre, même si le document EP-A-0 071 533 montre que la composition décrite permet de baisser le taux d'éthylène sur huit jours, rien n'est indiqué quant au comportement des végétaux au-delà de cette période.

Le document japonais JP-A-02106335 décrit un dispositif d'un support sur lequel sont disposés deux matières différentes. L'une est disposée sous forme d'un gel (slurry) contenant les particules absorbantes, l'autre est pulvérisée à la surface de la première couche et contient un agent irradiant. La deuxième couche empêche que les particules soient libres en dehors de la couche absorbante.

Dans JP-A-10337802 un dispositif constitué d'une plaque enduite d'une poudre de zéolite ou de charbon actif ou d'un mélange des deux est décrit. Une couche adhésive est placée sur les deux faces de la plaque. Ce document ne prévoit pas de maintenir une partie des particules en dehors de la couche absorbante.

Dès lors, le problème que se propose de résoudre l'invention est de fournir un dispositif destiné à lutter contre la dégradation des végétaux après récolte, nécessitant la mise en oeuvre d'une faible quantité de matière active par élimination de l'éthylène dégagé, tout en maintenant, voire en augmentant, l'efficacité à long terme de ladite matière par rapport aux solutions proposées dans l'art antérieur.

Un autre objectif de l'invention est d'assurer la conservation uniforme des végétaux lors de leurs stockage, et ce indépendamment de leur volume.

Pour ce faire, l'invention propose un dispositif destiné à lutter contre la dégradation des végétaux comprenant un mélange de poudres apte à adsorber, puis éliminer par rupture de la double liaison, l'éthylène dégagée par lesdits végétaux.

Ce dispositif se caractérise en ce qu'il se présente sous forme d'un support enduit d'une couche adhésive, dont la surface est recouverte dudit mélange de poudre, lequel se présente sous forme d'une pellicule de grains unitaires et individualisés.

Selon une caractéristique essentielle du dispositif, la poudre est déposée à la surface de la couche adhésive de sorte qu'une partie de la surface de chaque grain constitutif de la poudre ne soit pas incluse dans ladite couche adhésive mais en dehors de celle-ci. Il s'ensuit que chaque grain est rendu actif vis à vis de la dégradation de l'éthylène conduisant ainsi à diminuer considérablement la quantité de poudre nécessaire par rapport aux dispositifs de l'art antérieur. En outre, les grains sont déposés de sorte à former une très fine couche de grains unitaires, c'est à dire individualisés non superposés, l'épaisseur de la pellicule de grains correspondant sensiblement à la taille des grains. Cette caractéristique, ajoutée au fait que la répartition des grains sur le support est effectuée de sorte à être homogène, permet de disposer d'une surface, dont l'activité est optimale par rapport à la quantité de poudre déposée.

Le phénomène de dégradation est ralenti selon les mêmes principes que ceux décrits précédemment, à savoir principalement la fixation de l'éthylène sur une matière inerte, puis rupture de la double liaison du gaz, notamment par réaction d'oxydation.

Dès lors, pour permettre d'adsorber l'éthylène dégagé par le végétal, le mélange de poudres comprend une matière inerte choisie dans le groupe comprenant l'alumine activé, le charbon actif, l'argile, la dolomie, la zéolithe, les diatomées, la perlite, la bentonite, la kaolin activé, le dioxyde de titane seuls ou en mélange. Bien entendu, plus la surface spécifique de la matière inerte sera élevée et plus l'adsorption des gaz sera importante.

En outre, certaines des matière inertes choisies pour fixer l'éthylène présentent également un pouvoir oxydant. On notera en particulier le pouvoir oxydant de la dolomite vis à vis de l'éthylène dû à la quantité de manganèse qu'elle contient.

De même, pour permettre la fixation d'eau lorsque l'éthylène est dégradé par un agent oxydant, le mélange de poudres contient également un sel hygroscopique choisi dans le groupe comprenant le chlorure, le nitrate, le carbonate ou le sulfate de calcium, de magnésium, de sodium ou de potassium, les hydrures de calcium ou de magnésium et les polyphosphates, seuls ou en mélange.

Pour entraîner la rupture de la double liaison de l'éthylène, par oxydation, une fois que le gaz est fixé sur la matière adsorbante, le mélange de poudres contient également un agent oxydant choisi dans le groupe comprenant les sels de potassium, avantageusement le permanganate de potassium, les sels de sodium, les sels de manganèse, le tétroxyde d'osmium, seuls ou en mélange.

Pour favoriser la formation de dioxyde de carbone, lequel tend à diminuer le taux d'émission d'éthylène, le mélange de poudres comprend en outre du carbonate ou bicarbonate de sodium.

Par ailleurs, le Demandeur a constaté que, de façon tout à fait surprenante, l'incorporation dans la poudre de sels d'iode ou d'iode pur, seuls ou en mélange, permettait d'abaisser considérablement le taux d'éthylène dégagé.

Dès lors, l'invention concerne également une composition destinée à lutter contre la dégradation des végétaux, après récolte, par adsorption sur une matière inerte puis élimination de l'éthylène dégagé, caractérisée en ce qu'elle comprend des sels d'iode ou de l'iode pur, apte à rompre la double liaison de l'éthylène.

Dans une forme de réalisation avantageuse, la composition comprend entre 5 et 30 % en poids de sels d'iode ou d'iode pur.

Pour résoudre le problème de proposer un dispositif qui agisse de façon uniforme vis à vis de tous les fruits et légumes ou fleurs, notamment lors de leur stockage, le support choisi est un support fibreux ou un support non fibreux.

Dès lors, la souplesse du support permet d'emballer l'ensemble des végétaux à conserver, mettant ainsi le dispositif au contact direct ou à proximité proche de chacun des végétaux, en mettant en oeuvre un minimum de matière.

Parmi les supports fibreux, on choisit avantageusement les papiers pour emballage du type notamment papier cristal, papier sulfurisé et papier kraft.

Lorsque le support est un support non fibreux, il se présente sous forme d'un film plastique, dont le matériau constitutif est choisi dans le groupe comprenant le polyéthylène, le polypropylène, la cellophane, seuls ou en mélange.

Pour permettre de fixer la poudre active sur le support, le dispositif comprend en outre une couche adhésive comprenant un agent liant choisi dans le groupe comprenant l'amidon, la carboxyméthylcellulose, l'alcool polyvinylique, les homo-et copolymères émulsionnables du type acrylique, styrène, butadiene et dérivés, seuls ou en mélange.

Pour obtenir une adhésion efficace de la poudre, la concentration de l'agent liant dans la couche adhésive est comprise entre 1 et 20 %, avantageusement 5 % en matière sèche.

Pour une concentration inférieure à 1 %, la poudre n'adhère pas sur le support.

Pour une concentration supérieure à 20 %, on n'obtient pas d'effet supplémentaire.

Bien entendu, la couche adhésive est fabriquée de manière connue par mise en solution de l'agent liant dans un solvant approprié qui est choisi en fonction de la nature de l'agent liant.

En outre, la couche adhésive est enduite sur le support à raison de 0.5 à 15 g/m² en fonction de la nature de l'agent liant retenu, avantageusement 3 g/m² en sec.

Selon une autre caractéristique de l'invention, la poudre est déposée sur la couche adhésive à raison d'au moins 0,1 g/m² en sec, avantageusement 2 à 5 g/m².

Bien entendu, la masse de poudre à déposer sera choisie en fonction de l'activité et du volume de végétaux à traiter, le dépôt n'excédant pas 60 g/m².

Le procédé de fabrication de la poudre, tel qu'il est décrit dans l'art antérieur et consistant à simplement mélanger l'ensemble des poudres, n'est pas satisfaisant dans la mesure où il présente l'inconvénient de conduire à une poudre dont la taille et la répartition des grains dans la poudre finale ne sont pas homogènes. En conséquence, la quantité de poudre destinée à être déposée sur le support n'est pas optimale, puisqu'une grande partie de la surface active de la poudre peut être maintenue à l'intérieur de la couche adhésive. En outre, l'efficacité de la poudre n'est pas uniforme.

Pour résoudre ce problème, l'invention propose de fabriquer le mélange de poudres selon le procédé suivant qui consiste :
- à mélanger, sous agitation, les différents constituants du mélange de poudres avec de l'eau jusqu'à obtention d'une pâte ;
- puis, à extruder la pâte ainsi obtenue, à travers une filière ;
- à évaporer ensuite l'eau par séchage ;
- à broyer alors le produit obtenu jusqu'à obtention d'une poudre ;
- enfin, à calibrer la poudre par tamisage.

En pratique, la taille des grains constituant le mélange de poudres est comprise entre 20 et 500 micromètres, avantageusement 150 micromètres.

Le procédé de fabrication du dispositif de l'invention comprend les étapes suivantes :
- on enduit tout d'abord la couche adhésive sur le support ;
- puis, on dépose la poudre sur ladite couche adhésive de sorte à obtenir une fine pellicule de grains unitaires et individualisés répartis de façon homogène,
- enfin, on sèche le complexe obtenu de sorte à évaporer le solvant présent dans la couche adhésive.

L'enduction de la couche adhésive est effectuée par tous moyens connus du type lame d'air, barre égalisatrice ou lame lissante.

Par ailleurs, le dépôt de la poudre sur la couche adhésive est effectuée par procédé mécanique du type cylindre ou évent, rideau, pulvérisation par buses.

Dans tous les cas, les paramètres de fonctionnement des différents matériels seront déterminés de sorte à obtenir le dépôt d'une fine pellicule de poudre, dont les grains restent individualisés et non superposés.

Ainsi par exemple, dans le cas du cylindre rotatif dont le diamètre est compris entre 10 et 70 centimètres, avantageusement 50 centimètres, la vitesse de rotation du cylindre sera comprise entre 100 et 600 tours/minute, avantageusement 150 tours/minute.

Parallèlement, le support enduit de la couche adhésive défile sous le rideau de poudre à une vitesse comprise entre 100 et 200 mètres par minute, avantageusement 150 mètres par minute.

En pratique, le séchage est effectué à une température comprise entre 100 et 150° C, avantageusement 130° C pendant 1 à 30 secondes, avantageusement 5 secondes.

L'invention et les avantages qui en découlent ressortiront mieux de l'exemple de réalisation suivant.

La figure 1 est une représentation schématique de la structure du dispositif de l'invention.

La figure 2 est une représentation du cylindre rotatif mis en oeuvre pour le dépôt de la poudre.

La figure 3 (3a, 3b) est une représentation du dispositif de l'invention lorsqu'il est utilisé pour la conservation de roses.

Le dispositif fabriqué dans l'exemple suivant, comprend un support fibreux constituant un papier d'emballage. Ce papier d'emballage est destiné plus particulièrement à la conservation de roses coupées.

### 1/ Composition de la poudre

| | |
|---|---|
| Dolomie | 20 % en poids |
| Argile | 30 % en poids |
| NaCl | 20 % en poids |
| Iode pur | 30 % en poids |

### 2/ Fabrication de la poudre

Le mélange de poudre est préparé comme suit.

On mélange tout d'abord chacun des constituants avec de l'eau en quantité suffisante pour obtenir une pâte. La pâte est ensuite extrudée à travers une filière puis séchée jusqu'à évaporation complète de l'eau. Le produit obtenu est ensuite broyé, puis calibré à une taille de particules égale à 150 µm par tamisage.

### 3/ Fabrication du dispositif

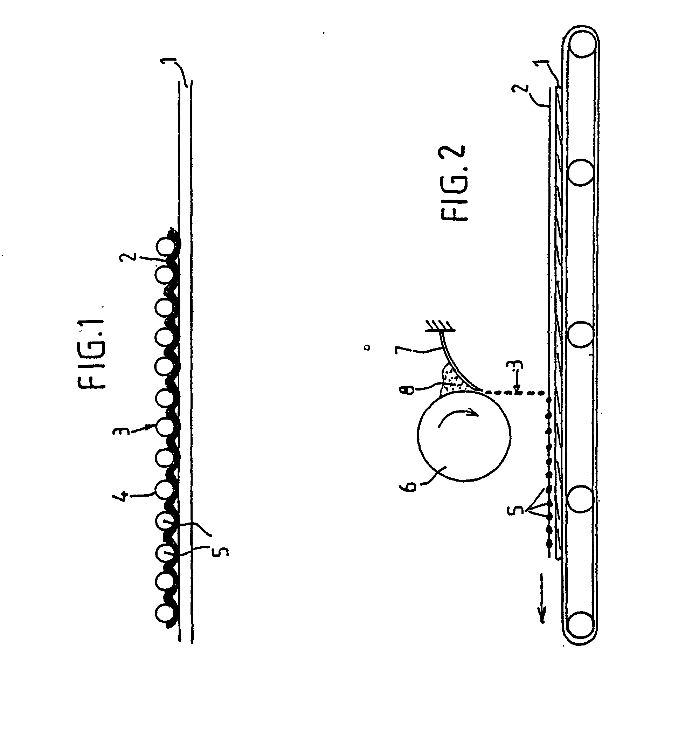

Sur la figure 1, on a représenté schématiquement la structure du dispositif, lequel comprend le support (1), la couche adhésive (2) et le mélange de poudres (3). Comme le montre cette figure, le mélange de poudres (3) est déposé sur la couche adhésive (2) de sorte que la partie (4) de la surface des grains (5) ne soient pas comprise dans la couche adhésive (2). En outre, les grains sont individualisés et non superposés conduisant à une pellicule dont l'épaisseur est égale à la taille des grains. Il s'ensuit que l'activité du dispositif est élevée malgré la faible quantité de poudre requise.

Le dispositif de la figure 1 est obtenu par le procédé suivant.

Sur un support composé de 100 % de fibres cellulosiques désigné papier CRISTAL commercialisé par AHLSTROM, on enduit par lame d'air, une couche adhésive, dont la concentration en agent liant (alcool polyvinylique) est égale à 10% en matière sèche. L'enduction est réalisée à raison de 3 g/m² en sec, soit 30 µm d'épaisseur.

On procède ensuite au dépôt de la poudre sur le support (1) enduit de la couche adhésive (2).

Pour ce faire, un cylindre métallique (6) muni d'une lame (7) est positionné au dessus du support enduit. Plus précisément, la lame (7) vient en appui contre le cylindre (6) formant ainsi un réservoir (8) de la poudre. La vitesse de rotation du cylindre, la contrainte de la lame sur le cylindre et la vitesse de défilement du support enduit sont réglés de sorte à obtenir sur le support le dépôt d'une fine pellicule de grains individualisés unitaires et non superposés.

Dans le présent exemple, le diamètre du cylindre métallique est égal à 50 centimètres, tandis que la lame présente une épaisseur de 0.25 mm pour une longueur de 8 cm. La vitesse de rotation du cylindre est de 150 tours/minute et la vitesse de défilement du support enduit est égale à 150 mètres/minute. Le dépôt de poudre est effectué à raison de 3 g/m².

Le complexe obtenu est ensuite séché dans un four à 130° C pendant 5 secondes.

### 4/ Conservation des roses

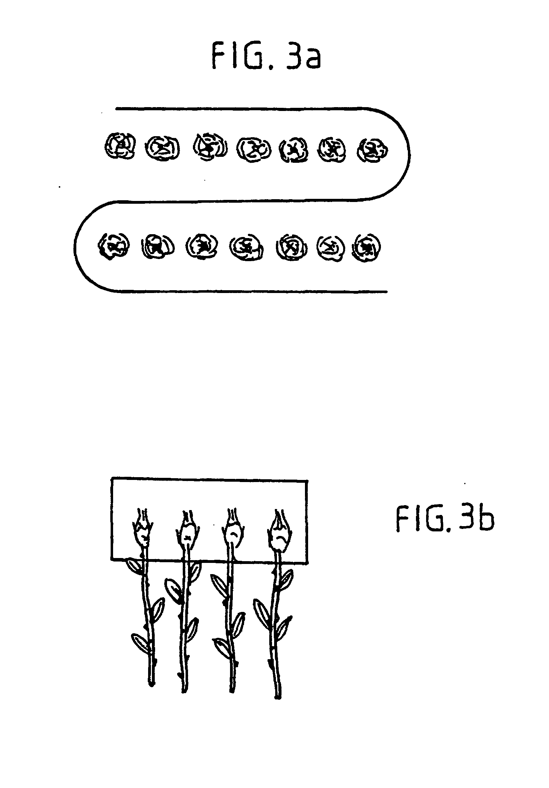

8 000 roses en bouton ont été cueillies en Amérique Centrale. Les 8000 roses ont été empaquetées par containers de 300 constitué 10 lots de 30 à l'aide d'une bande de papier fabriquée selon le procédé précédemment décrit d'une longueur de 1 mètre et de 25 centimètres de largeur. Le papier d'emballage est maintenu à proximité des boutons comme le montre la figure 3a (fleurs de face) et 3b (fleurs de profil). Comme le montrent les figures, toutes les rosés sont positionnées à proximité du dispositif de sorte que le traitement est uniforme.

Après 6 jours de transport entre l'Amérique Centrale et la France, les boutons des rosés sont restés identiques, la tige est encore dure et les feuilles ne sont pas flétries. Les rosés sont ensuite maintenues à l'air libre à une température de 25° C. Au bout de 3 semaines, aucun signe de dégradation n'est visible.

En outre, on observe que le processus de vieillissement de la rosé est retardé. En effet, alors qu'il débute au 10^{ème} jour pour une rose normale, il est retardé au 35^{éme} jour pour une rose traitée par le dispositif de l'invention.

L'invention et les avantages qui en découlent ressortent bien de la description qui précède.

On notera notamment la faible quantité de poudre utilisée pour la fabrication du dispositif conduisant ainsi à diminuer considérablement le coût du traitement de conservation.

En outre, on note que la durée de conservation est très longue, de même que le phénomène de maturation est ralenti.

Enfin, la conservation des végétaux est uniforme dans des conditions de stockage normales.

## Revendications

1. Dispositif destiné à lutter contre la dégradation des végétaux comprenant un mélange de poudres apte à adsorber, puis éliminer par rupture de la double liaison, l'éthylène dégagée par lesdits végétaux, **caractérisé en ce qu'**il se présente sous forme d'un support enduit d'une couche adhésive, dont la surface est recouverte dudit mélange de poudre, lequel se présente sous forme d'une pellicule de grains unitaires et individualisés, dont une partie de la surface reste en dehors de la couche adhésive, la poudre étant déposée sur la couche adhésive à raison de 0,1 à 60 g/m² en sec.

2. Dispositif selon la revendication 1, **caractérisé en ce que** le mélange de poudres comprend une matière inerte choisie dans le groupe comprenant l'alumine activé, le charbon actif, l'argile, la dolomite, la zéolithe, les diatomées, la perlite, la bentonite, la kaolin activé, le dioxyde de titane, seuls ou en mélange.

3. Dispositif selon l'une des revendications précédentes, **caractérisé en ce que** le mélange de poudres contient un sel hygroscopique choisi dans le groupe comprenant le chlorure, le nitrate, le carbonate ou le sulfate de calcium, de magnésium, de sodium ou de potassium, les hydrures de calcium ou de magnésium et les polyphosphates, seuls ou en mélange.

4. Dispositif selon l'une des revendications précédentes, **caractérisé en ce que** le mélange de poudres contient également un agent oxydant choisi dans le groupe comprenant les sels de potassium, les sels de sodium, les sels de manganèse, le tétroxyde d'osmium, seuls ou en mélange.

5. Dispositif selon l'une des revendications précédentes, **caractérisé en ce que** le mélange de poudres comprend en outre du carbonate ou bicarbonate de sodium.

6. Dispositif selon l'une des revendications précédentes, **caractérisé en ce que** le mélange de poudres comprend en outre des sels d'iode ou de l'iode pur.

7. Dispositif selon l'une des revendications précédentes, **caractérisé en ce que** le support est un support fibreux ou un support non fibreux.

8. Dispositif selon la revendication 7, **caractérisé en ce que** le support fibreux est choisi dans le groupe comprenant les papiers pour emballage du type notamment papier cristal, papier sulfurisé et papier kraft.

9. Dispositif selon la revendication 7, **caractérisé en ce que** le support non fibreux se présente sous forme d'un film plastique, dont le matériau constitutif est choisi dans le groupe comprenant le polyéthylène, le polypropylène, la cellophane, seuls ou en mélange.

10. Dispositif selon l'une des revendication précédentes, **caractérisé en ce que** la couche adhésive comprend un agent liant choisi dans le groupe comprenant l'amidon, la carboxyméthylcellulose, l'alcool polyvinylique, les homo- et copolymères émulsionnables du type acrylique, styrène, butadiene et dérivés, seuls ou en mélange.

11. Dispositif selon la revendication 10, **caractérisé en ce que** la concentration de l'agent liant dans la couche adhésive est comprise entre 1 et 20 %, avantageusement 5 %, en matière sèche.

12. Dispositif selon l'une des revendications 10 ou 11, **caractérisé en ce que** la couche adhésive est enduite sur le support à raison de 0.5 à 15 g/m², avantageusement 3 g/m² en sec.

13. Dispositif selon l'une des revendications précédentes, **caractérisé en ce que** la poudre est déposée sur la couche adhésive à raison d'au moins 0,1 g/m² en sec, avantageusement 2 à 5 g/m².

14. Dispositif selon l'une des revendications précédentes, **caractérisé en ce que** la taille des grains constituant le mélange de poudres est comprise entre 20 et 500 micromètres, avantageusement 150 micromètres.

15. Procédé pour la fabrication du dispositif objet de l'une des revendications 1 à 14, **caractérisé en ce qu'**il comprend les étapes suivantes:
• on enduit tout d'abord la couche adhésive sur le support ;
• puis, on dépose le mélange de poudres sur ladite couche adhésive, de sorte à obtenir une fine pellicule de grains unitaires et individualisés répartis de façon homogène.
• enfin, on sèche le complexe obtenu de sorte à évaporer le solvant présent dans la couche adhésive.

16. Procédé selon la revendication 15, **caractérisé en ce que** la fabrication du mélange de poudres consiste :
• à mélanger, sous agitation, les différents constituants du mélange de poudres avec de l'eau jusqu'à obtention d'une pâte ;
• puis, à extruder la pâte ainsi obtenue, à travers une filière;
• à évaporer ensuite l'eau par séchage ;
• à broyer alors le produit obtenu jusqu'à obtention d'une poudre ;
• enfin, à calibrer la poudre par tamisage.

17. Procédé selon la revendication 15,**caractérisé en ce que** le dépôt de la poudre est effectué au moyen d'un cylindre muni d'une lame formant réservoir de poudre, le diamètre du cylindre étant compris entre 10 et 70 centimètres, avantageusement 50 centimètres, la vitesse de rotation du cylindre étant comprise entre 100 et 600 tours par minute, avantageusement 150 tours par minute.

18. Procédé selon la revendication précédente, **caractérisé en ce que** le support enduit de la couche adhésive défile sous le rideau de poudre à une vitesse comprise entre 100 et 200 mètres par minute, avantageusement 150 mètres par minute.

## Patentansprüche

1. Vorrichtung zur Bekämpfung des Verderbens von Pflanzen mit einer Pulvermischung, die zur Adsorption und anschließenden Eliminierung durch Aufbrechen der Doppelbindung des durch die genannten Pflanzen freigesetzten Ethylens geeignet ist, **dadurch gekennzeichnet, daß** sie die Form eines Trägers aufweist, der mit einer haftenden Schicht beschichtet ist, deren Oberfläche mit der genannten Pulvermischung bedeckt ist, die die Form einer Haut aus einheitlichen und vereinzelten Körnern aufweist, von denen ein Teil der Oberfläche außerhalb der haftenden Schicht verbleibt, wobei das Pulver auf der haftenden Schicht in der Menge von 0,1 bis 60 g/m² Trockengewicht aufgebracht ist.

2. Vorrichtung nach Anspruch 1, **dadurch gekennzeichnet, daß** die Pulvermischung ein inertes Material aufweist, welches aus der Gruppe ausgewählt ist, die aktiviertes Aluminium, Aktivkohle, Ton, Dolomit, Zeolith, Diatomeen, Perlit, Bentonit, aktiviertes Kaolin, Titandioxid, einzeln oder gemischt enthält.

3. Vorrichtung nach einem der vorangehenden Ansprüche, **dadurch gekennzeichnet, daß** die Pulvermischung ein hygroskopisches Salz enthält, welches aus der Gruppe ausgewählt ist, die Kalzium-, Natrium- oder Kaliumchlorid, -nitrat, -karbonat oder -sulfat, Kalzium- oder Magnesiumhydride und Polyphosphate, einzeln oder gemischt enthält.

4. Vorrichtung nach einem der vorangehenden Ansprüche, **dadurch gekennzeichnet, daß** die Pulvermischung ebenfalls ein Oxidationsmittel enthält, welches aus der Gruppe ausgewählt ist, die Kaliumsalze, Natriumsalze, Mangansalze, Osmiumtetroxid, einzeln oder gemischt enthält.

5. Vorrichtung nach einem der vorangehenden Ansprüche, **dadurch gekennzeichnet, daß** die Pulvermischung ferner Natriumkarbonat oder - bikarbonat enthält.

6. Vorrichtung nach einem der vorangehenden Ansprüche, **dadurch gekennzeichnet, daß** die Pulvermischung ferner Jodsalze oder reines Jod enthält.

7. Vorrichtung nach einem der vorangehenden Ansprüche, **dadurch gekennzeichnet, daß** der Träger ein faseriger Träger oder ein nicht-faseriger Träger ist.

8. Vorrichtung nach Anspruch 7, **dadurch gekennzeichnet, daß** der faserige Träger aus der Gruppe ausgewählt ist, die Packpapier und insbesondere Pergamin, Pergamentersatz und Kraftpapier enthält.

9. Vorrichtung nach Anspruch 7, **dadurch gekennzeichnet, daß** der nichtfaserige Träger die Form eines Kunststoffilms aufweist, der aus einem Material besteht, das aus der Gruppe ausgewählt ist, die Polyethylen, Polypropylen, Zellophan, einzeln oder gemischt enthält.

10. Vorrichtung nach einem der vorangehenden Ansprüche, **dadurch gekennzeichnet, daß** die haftende Schicht ein Bindemittel enthält, das aus der Gruppe ausgewählt ist, die Stärke, Karboxymethylzellulose, Polyvinylalkohol, emulgierbare Homo- und Copolymere des Typs Acryl, Styrol, Butadien und deren Derivate, einzeln oder gemischt enthält.

11. Vorrichtung nach Anspruch 10, **dadurch gekennzeichnet, daß** die Konzentration des Bindemittels in der haftenden Schicht zwischen 1 und 20%, vorteilhafterweise 5%, des trockenen Materials beträgt.

12. Vorrichtung nach einem der Ansprüche 10 oder 11, **dadurch gekennzeichnet, daß** die haftende Schicht auf dem Träger in einer Menge von 0,5 bis 15 g/m², vorteilhafterweise 3 g/m², Trockengewicht aufgetragen ist.

13. Vorrichtung nach einem der vorangehenden Ansprüche, **dadurch gekennzeichnet, daß** das Pulver auf der haftenden Schicht in einer Menge von mindestens 0,1 g/m² Trockengewicht, vorteilhafterweise 2 bis 5 g/m², aufgetragen ist.

14. Vorrichtung nach einem der vorangehenden Ansprüche, **dadurch gekennzeichnet, daß** die Größe der die Pulvermischung bildenden Körner zwischen 20 und 500 Mikrometern, vorteilhafterweise 150 Mikrometer, beträgt.

15. Verfahren zur Herstellung einer Vorrichtung gemäß einem der Ansprüche 1 bis 14, **dadurch gekennzeichnet, daß** es die folgenden Schritte umfaßt:
• zunächst Beschichten des Trägers mit der haftenden Schicht;
• anschließend Aufbringen der Pulvermischung auf die genannte haftende Schicht, so daß eine feine Haut aus einheitlichen und vereinzelten, homogen verteilten Körnern erhalten wird;
• schließlich Trocknen des erhaltenen Gebildes, so daß das in der adhäsiven Schicht vorhandene Lösungsmittel verdampft wird.

16. Verfahren nach Anspruch 15, **dadurch gekennzeichnet, daß** die Herstellung der Pulvermischung folgendes umfaßt:
• Mischen unter Rühren der verschiedenen Bestandteile der Pulvermischung mit Wasser, bis eine Paste erhalten wird;
• anschließend Extrudieren der so erhaltenen Paste durch eine Düse;
• anschließend Verdampfen des Wassers durch Trocknung;
• Zerstoßen des erhaltenen Produktes, bis ein Pulver erhalten wird;
• schlieBlich Aussortieren des Pulvers durch Siebung.

17. Verfahren nach Anspruch 15, **dadurch gekennzeichnet, daß** das Aufbringen des Pulvers mittels eines Zylinders durchgeführt wird, der mit einer Klinge versehen ist, die ein Pulverreservoir bildet, wobei der Durchmesser des Zylinders zwischen 10 und 70 Zentimetern, vorteilhafterweise 50 Zentimeter, beträgt und die Drehgeschwindigkeit des Zylinders zwischen 100 und 600 Umdrehungen pro Minute, vorteilhafterweise 150 Umdrehungen pro Minute, beträgt.

18. Verfahren nach dem vorangehenden Anspruch, **dadurch gekennzeichnet, daß** der mit der haftenden Schicht beschichtete Träger unterhalb des Pulvervorhangs mit einer Geschwindigkeit zwischen 100 und 200 Metern pro Minute, vorteilhafterweise 150 Meter pro Minute, hindurchtritt.

## Claims

1. A device for protecting plants against decay, comprising a mixture of powders capable of adsorbing, then eliminating, by breading of the double bond, the ethylene released by said plants, which is in the form of a substrate coated with an adhesive layer, whose surface is covered with said powder mixture, which is in the form of a film of single-unit and individualized grains, of which part of the surface remains outside the adhesive layer, the powder being deposited on the adhesive layer in an amount of 0,1 to 60 g/m² on a dry basis.

2. The device as claimed in claim 1, **characterized in that** the mixture of powders comprises an inert material chosen from the group comprising activated alumina, activated charcoal, clay, dolomite, zeolite, diatoms, perlite, bentonite, activated kaolin, titanium dioxide, alone or as a mixture.

3. The device as claimed in one of the preceding claims, **characterized in that** the mixture of powders contains a hygroscopic salt chosen from the group comprising calcium, magnesium, sodium or potassium chloride, nitrate, carbonate or sulfate, calcium or magnesium hydrides, and polysulfates, alone or as a mixture.

4. The device as claimed in one of the preceding claims, **characterized in that** the mixture of powders also contains an oxidizing agent chosen from the group comprising potassium salts, sodium salts, manganese salts, osmium tetroxide, alone or as a mixture.

5. The device as claimed in one of the preceding claims, **characterized in that** the mixture of powders comprises, in addition, sodium carbonate or bicarbonate.

6. The device as claimed in one of the preceding claims, **characterized in that** the mixture of powders comprises, in addition, iodine salts or pure iodine.

7. The device as claimed in one of the preceding claims, **characterized in that** the substrate is a fibrous substrate or a nonfibrous substrate.

8. The device as claimed in claim 7, **characterized in that** the fibrous substrate is chosen from the group comprising packaging paper in particular of the glassine, parchment paper and kraft paper type.

9. The device as claimed in claim 7, **characterized in that** the nonfibrous substrate is in the form of a plastic film whose constituent material is chosen from the group comprising polyethylene, polypropylene, cellophane, alone or as a mixture.

10. The device as claimed in one of the preceding claims, **characterized in that** the adhesive layer comprises a binding agent chosen from the group comprising starch, carboxymethylcellulose, polyvinyl alcohol, emulsifiable homo- or copolymers of the acrylic, styrene and butadiene type and derivatives, alone or as a mixture.

11. The device as claimed in claim 10, **characterized in that** the concentration of binding agent in the adhesive layer is between 1 and 20 %, advantageously 5 %, as dry matter.

12. The device as claimed in either of claims 10 and 11, **characterized in that** the adhesive layer is coated on the substrate in an amount of 0,5 to 15 g/m², advantageously 3 g/m² on a dry basis.

13. The device as claimed in one of the preceding claims, **characterized in that** the powder is deposited on the adhesive layer in an amount of at least 0,1 g/m² on a dry basis, advantageously 2 to 5 g/m².

14. The device as claimed in one of the preceding claims, **characterized in that** the size of the grains constituting the mixture of powders is between 20 and 500 micrometers, advantageously 150 micrometers.

15. A method for making the device which is the subject of one of claims 1 to 14, **characterized in that** it comprises the following steps:
- the adhesive layer is first of all coated onto the substrate;
- then, the powder mixture is deposited on said adhesive layer so as to obtain a fine film of homogeneously distributed single-unit and individualized grains,
- finally, the complex obtained is dried so as to evaporate the solvent present in the adhesive layer.

16. The process as claimed in claim 15, **characterized in that** the making of the mixture of powders consists:
- in mixing, with stirring, the various constituents of the mixture of powders with water until a paste is obtained;
- then, in extruding the paste thus obtained, through a die;
- in then evaporating the water by drying;
- in then grinding the product obtained until a powder is obtained;
- finally, in calibrating the powder by sieving.

17. The method as claimed in claim 15, **characterized in that** the powder is deposited by means of a cylinder equipped with a blade forming a powder reservoir, the diameter of the cylinder being between 10 and 70 centimeters, advantageously 50 centimeters, the speed of rotation of the cylinder will be between 100 and 600 revolutions/minute, advantageously 150 revolutions/minute.

18. The method as claimed in the preceding claim, **characterized in that** the substrate coated with the adhesive layer passes under the powder curtain at a speed of between 100 and 200 meters per minute, advantageously 150 meters per minute.
